# EUROPÄISCHE PATENTANMELDUNG

(11) **EP 2 236 110 A2**
(43) Veröffentlichungstag der Anmeldung: **06.10.2010**
(21) Anmeldenummer: 10002541.0
(22) Anmeldetag: 11.03.2010
(51) Int. Cl.: A61F 13/15, A61F 13/20, A61F 13/511

(54) **Hüllvlies für einen Tampon**

(30) Priorität: 31.03.2009 DE 102009015127
(71) Anmelder: Sandler AG, 95126 Schwarzenbach/Saale (DE)
(72) Erfinder:

(57) **Zusammenfassung**

Die vorliegende Erfindung handett von einem Hüllvlies (1) für absorbierende Tampons, umfassend eine im späteren Gebrauch dem Körper zugewandte Seite (2) und eine im späteren Gebrauch einem Saugkörper (4) zugewandten Seite (3) **dadurch gekennzeichnet, dass** die Seite (2) aus einem nach einem Trockenverfahren gebildeten Vliesstoff besteht und dass die die Seite (3) aus einem Extrusionsvliesstoff besteht.

## Beschreibung

### Stand der Technik

Die vorliegende Erfindung betrifft Hüllvliesstoffe für Tampons die für die Damenhygiene eingesetzt werden.

Tampons der gattungsgemäßen Art bestehen aus
einem Hüllvlies,
einem absorbierenden Kern
und einem Rückholladen.

Das Hüllvlies hat dabei mehrere Funktionen: beispielsweise muss es das leichte Einführen des Tampons zu gewährleisten, muss Körpeflüssigkeiten zum absorbierenden Kern hin passieren lassen, muss abstehende Fasern des absorbierenden Kerns zurückhalten und es darf sich gegen Ende der Gebrauchszeit nicht vom absorbierenden Kern anlösen.

In der Vergangenheit gab es verschiedene Ansätze diese, sich zum Teil widersprechenden Funktionen zu kombinieren.

Üblicherweise wird bei der Herstellung von Tampons ein Kardenband aus Viskose oder Mischungen Viskose und Baumwolle mit einem Hüllvlies aus synthetischen Fasern im Heißsiegelverfahren miteinander verbunden. Dabei werden beispielsweise Vlieskonstruktionen aus bikomponenten Stapelfasern aus Polyethylen im Mantel und Polypropylen oder Polyethylenterephthalat im Kern eingesetzt. Während der sogenannten Versiegelung, dem Verbinden des Hüllvlieses mit dem absorbierenden Kern wird die niedrigschmelzende Mantelkomponente zum Verkleben genutzt. Für ein optimales Einführen des Tampons wird weiterhin ein besonders glattes Hüllvlies bevorzugt. Solche Produkte, beschrieben in DE 19753665 C2, zeigen schlechtere Versiegeleigenschaften zum Kardenband, da durch die vorhergehende Glättung dieses Vlieses die Kernkomponente dieser Faser oftmals nicht mehr zentrisch von der Mantelkomponente umschlossen wird und freillegt. Freiliegende Kernkomponente ist aber nicht siegelfähig, somit ist partiell eine nur ungenügende Haftung zwischen Hüllvlies und absorbierenden Kern die Folge.

Weiterhin sind aus der EP 1194100 B1 Produkte bekannt, bei welchen zur Erzielung einer glatten Oberfläche perforierte Lochfolien als Hüllschicht eingesetzt werden. Nachteilig ist dabei, die Tatsache, das bei der Verarbeitung besondere Siegelwerkzeuge notwendig sind, um das Verschließen der Löcher zu verhindern. Aufgrund der im Umhüllungsmaterial vorhandenen Perforierungen reduziert sich die Kontaktfläche zwischen dem Umhüllungsmaterial und dem Tamponkern, wodurch die Gefahr besteht, dass keine ausreichende Verbundhaftung erreicht wird und das durch die Perforationen Faser des absorbierenden Kerns migrieren können.

In EP 0401358 B1 ist ein Tampon dargestellt, der eine einschichtige Tamponumhüllung aus Fasern umfasst, die mit einem Pulverbindemittel thermisch miteinander verbunden und auf einen als Tamponkern fungierenden absorbierenden Wattebausch aufgepresst sind. Der Tamponkern besteht aus einem Gemisch von Viskosefilamentfasem und Baumwolle.

Nachteilig dabei ist jedoch der aufwendige Herstellungsprozess, da hierbei eine zusätzliche, präzise Pulverdosierung sowie der kostenintensive Einsatz von Pulverbindemittel erforderlich ist. Eine zu gering eingebrachte Pulvermenge führt dazu, dass die Weichheit zu stark erhöht und die Festigkeit des Vlieses zu niedrig ist, während eine zu viel eingebrachte Pulvermenge einen zu steifen Warenausfall bewirkt, woraus letztendlich eine unzufriedenstellende Saugleistung des Tampons resultiert. Zudem besteht beim Produktionsprozess der Pulverbindung das Problem, dass das Pulver absolut homogen in den Faserverband eingebracht werden muss, d. h., ein ungenügendes bzw. nur partielles Verschmelzen des Pulverbindemittels und eine damit einhergehende zu schwache Verfestigung kann bei der Tamponherstellung mit hoher Verarbeitungsgeschwindigkeit zum Auftrennen oder Zerreißen der Tamponumhüllung führen.

Die DE 202008006229 U1 behandelt Hüllvliesstoffe für Tampon in der Frauenhygiene. Als Abdeckmaterial dient ein thermisch verfestigter Vliesstoff aus bikomponenten Filamentvliesstoffen. Die Erfinder haben dabei jedoch nicht erkannt, dass für die erforderliche Gewährleistung des Tragekomforts während der gesamten Tragezeit und zur Vermeidung der Delamination beim Entfernen des Tampons eine Verbesserung der Haftkraft zwischen Tamponkern und Abdeckvliesstoff von absoluter Relevanz ist. Somit besteht auch bei derartigen Tampons nach wie vor die Gefahr, dass sich der Abdeckvliesstoff beim Entnehmen des Tampons aus der Körperhöhle ablöst und darin verbleibt.

Die EP 0685213 A2 beschreibt das in-situ Aufbringen einer Hüllschicht aus thermopiastischen Faser und / oder Filamenten direkt auf den auf den absorbierenden Kern des späteren Tampons. Beim späteren thermischen Verpressen der Kombination aus Hüllschicht und absorbierenden Kern, schmilzt die Hüllschicht und verbindet sich mit dem absorbierenden Kern.
Nachteilig ist dabei, dass die Porosität der Hüllschicht nach dem thermischen Verpressen nicht genau vorbestimmbar ist, somit ein Teil der Oberfläche für Körperflüssigkeiten undurchdringbar wird.

Aufgabe der Erfindung war es daher, ein Hüllvlies (1) für einen Tampon (5) oereitzustellen, welches die Nachteile des Standes der Technik zu vermeidet.

### Detaillierte Beschreibung der Erfindung

Die Lösung der Aufgabe geschieht anhand der Merkmale der Ansprüche.

Das erfindungsgemäße Hüllvlies (1) besteht aus einer Kombination von nach Trockenverfahren hergestellten Vliesstoffen, die auf der im späteren Gebrauch dem Körper zugewandten Seite (2) zu liegen kommen, mit Feinfaser-Spinnvlicsstoffen, die auf der im späteren Gebrauch der Saugschicht zugewandten Seite (3) angeordnet sind.

Unter nach einem Trockenverfahren hergestellten Vliesstoffen sind gemäß der Einteilung auf Seite XI im Buch "Vliessloffe", Wiley VCH Verlag, D-69469 Weinheim, erschienen im Jahr 2000, Faservliesstoffe und Extrusionsvliesstoffe zu verstehen. Erfindungsgemäß bestehen die nach einem Trockenverfahren hergestellten Vliesstoffe aus thermoplastischen Stapelfasern oder Filamenten mit einem Durchmesser von > 10µm. Gängige Verfahren zur Herstellung derartiger Vliesstoffe können dem Buch "Vliesstoffe" auf den Seiten 139-228 entnommen werden.

Der Begriff Feinfaser-Spinnvliesstoff wird im Sinne dieser Erfindung für Vliesstoffe verwendet, die aus thermoplastischen Polymeren bestehen, einen Faserdurchmesser von <10µm aufweisen und nach einem Schmelzbias- oder Elektrostatik-Spinnviiesverfahren hergestellt werden. Die prinzipiellen Verfahren dazu sind im Buch "Vliesstoffe" auf den Seiten 219-222 erläutert.

Für die Herstellung des erfindungsgemäßen Hüllvlieses (1) wird beispielsweise zunächst der die spätere Seite (2) bildende Vliesstoff unter Verwendung eines Trockenverfahrens vorgefertigt.

Bevorzugt zur Anwendung kommen dabei Filamentspinnvliesstoffe und besonders bevorzugt Faservliesstoffe aus Stapelfasern. Diese Vliesstoffe können sowohl aus monokomponenten als auch aus bikomponenten Faserstoffen bzw Filamenten gebildet werden und können, je nach Verfahrensablauf verfestigt, beispielweise durch Kalandrieren oder Heißluftbinden aber auch, für den Fall eines Spinnvlieses innerhalo einer Spinnvlies - Meltblown-Anlage, zunächst unverfestigt sein.

für die die Seite (2) bildenden Fasern werden Faserdurchmesser > 10µm eingesetzt beziehungsweise bei Filarnentspinnvliesstoffen erzeugt, wobei sich als besonders geeignet Durchmesser von 14-25µm gezeigt haben.

Zum Einsatz können dabei alle thermoplastischen Fasern kommen, es sind aber auch Mischungen von thermoplastischen mit nicht-thermoplastischen Fasern, beispielsweise Viskose denkbar.

In den nachfolgenden Beispielen wird für den die spätere Seile (2) bildenden Vliesstoff ein kardierter, thermisch kalanderverfestigter Stapelfaservliesstoff verwendet, welcher aus 100% bikomponenten Kern-Mantel-Stapelfasern aus Polyethylenterephthalat im Kern und Polyethylen als Mantel in einem Volumenverhältnis von 50% zu 50% besteht. Der Faserdurchmesser dieser zylindrischen extrudierten Faser beträgt 14µm, entsprechend einem Fasertiter von 2.2dtex, die Stapellänge liegt bei 51mm. Aus diesen Fasern wird zunachst ein Faserflor hergestellt und anschließend mittels eines beheizten, gravierten Kalanders verfestigt. Dabei kommt eine Pressflächen von 23% und eine Temperatur von 135°C zum Einsatz, das Flächengewicht liegt bei 12 g/qm.

Dieser, die spätere Seite (2) bildende Vliesstoff wird nun einer Schmelzblaseinrichtung zugeführt und mit einem Feinfaser-Spinnviiesstoff beschichtet, der die Seite (3) des erfindungsgemäßen Hüllvliesstoffes bildet. Dabei kommen übliche Schmelzblas-, aber auch Elektrostatik-Spinnvlies-Verfahren zum Einsatz.

Für die Herstellung des Feinfaserspinnvliesstoffs werden Granulate bzw Polymere verwendet, die einen möglichst hohen Schmelzflussindex bei vergleichsweise geringen Schmelzpunkten aufweisen.

Die Verfahreneinstellung für die Herstellung der die Seite (3) bildenden Feinfasern ist so zu wählen, das sich ein mittlerer Faserdurchmesser von <10µm ergibt, bevorzugt ist dabei eine Verfahrensführung, welche Feinfasern mit einem mittleren Durchmesser von kleiner 5µm erzeugt.

Bevorzugt sind Granulate aus Polypropylen mit einem Schmelzflussindex von größer 300 g/10min, gemessen bei 2.16kg und 230°C gemäß DIN 1133. Es ist aber auch die Verwendung anderer Granulate oder modifizierter Polymere denkbar, deren Schmelzverhalten durch Zusätze zum Granulat erst im Extruder erhalten wird.

In den nachfolgenden Beispielen wird ein Polypropylen-Granulat mit einem Schmelzflussindex von ca 1200g/10min verwendet.

Das Flächengewichtsverhältnis der Seiten (2) und (3) ist erfindungsgemäß so zu wählen, dass die Seite (3) maximal den gleichen Gewichtsanteil am Gesamtflächengewicht ausmacht. Bevorzugt ist dabei ein Verhältnis von 3:1 bis 6:1. in den nachfolgenden Beispielen hat das die Seite (2) bildenden Vllies ein Flächengewicht von 12g/qm, das die Seite (3) bildende ein Flächengewicht von 3g/qm, das Flächendewichtsvorträltnis von Seite(2) zu Seite (3) liegt daher bei 4:1. Höhere Anteile von Seite (3), d.h. Verhältnisse von 1:2 oder größer sind nicht dienlich. da dann die Quellung des Tampons im späteren Gebrauch eingeschränkt wird. Verhältnisse von kleiner 15:1 behindern zwar nicht die Quellung, jedoch ist dann die Haftung des Hüllvlieses auf dem Saugkern nicht ausreichend.

Die Verbindung der die beiden Seiten des erfindungsgemäßen Hüllvlieses (1) bildenden Vliese geschieht vorzugweise durch mechanische Verstnckung der beiden Lagen während der Applikation des Feinfaser-Spinnvliesstoffes.

Abhängig von der Verfahrensführung kann aber auch eine Schmelzverbindung der den Feinfaser-Spinnvliesstoff bildenden Fasern mit den Fasern des Vliesstoffes der Seite (2) erfolgen, sofern diese Feinfasern noch in schmelzflüssigen Zustand auftreffen.

Die Verbindung der beiden Lagen kann auch durch eine Kalanderbehandlung bei Temperaturen zwischen 80 und 50°C, Kalanderdrücken von 20-80daN/cm und einem Pressflächenanteil von 4-30% unterstützt werden.

Als Saugkörper wird bei der Herstellung von Tampons üblicherweise ein Kardenband aus Viskose oder Mischungen Viskose und Baumwolle gebildet. Das Kardenband besteht beispielsweise aus 80% Viskosefasern, 1,7dtex / 40mm Stapellänge und 20 % Viskose trilobal 3.3 dtex / 40mm Stapellänge. Es hat dabei eine Breite von 50mm und ein Längengewicht von 8 g/m.

Bei der Herstellung eines Tampons (5) unter Verwendung des erfindungsgemäßen Hüjlvlieses (1) wird beispielsweise zunächst ein 50mm breiter und 125mm langer Abschnitt des Hüllvlieses (1) mit der Seite (3) auf das den späteren Saugkörper (4) bildende Kardenband abgelegt. In einem ersten Schritt wird das Hüllvlies (1) mittels einer beheizten, gravierten Presswalze auf das Kardenband über eine Länge von 85mm so angeheftet, dass das Hüllvlies (1) am Ende des Kardenband-Abschnittes 30-40mm übersteht.

Der so entstandene Zuschnitt mit einem Rückholfaden (6) versehen und zu einem Tamponrohling aufgewickelt, sodass im Inneren der Saugkörper (4) liegt und dieser von dem Hüllvlies (1) umhüllt wird. Dabei liegt die Seite (2) des Hüllvlieses (1) nach außen, die Seite (3) des Hüllvlieses (1) zum Saugkörper (4) hin.

Der so mit dem erfindungsgemäßen Hüllvlies (1) abgedeckte Tamponrohling wird nun dem endgültigen Versiegelungsprozess zugeführt. Dabei wir der Rohling in die spätere Form gepresst. Durch gleichzeitig anliegende Temperatur schmilzt bei dem erfindungsgemäßen Hüllvlies (1) nur das die Seite (3) bildende Polymer und verbindet sich aufgrund des hohen Schmelzflussindexes intensiv mit dem Saugkörper (4).

Da beim erfindungsgemäßen Hüllvlies (1) nur die Seite (3) zur Verbindung des Hüllvlieses (1) mit dem Saugkörper (4) genutzt wird, bleibt die Durchlässigkeit gegenüber Körperflüssigkeiten, die hauptsächlich von dem die Seile (2) bildenden Vliesstoff abhängt, weitgehend unbeeinflusst.

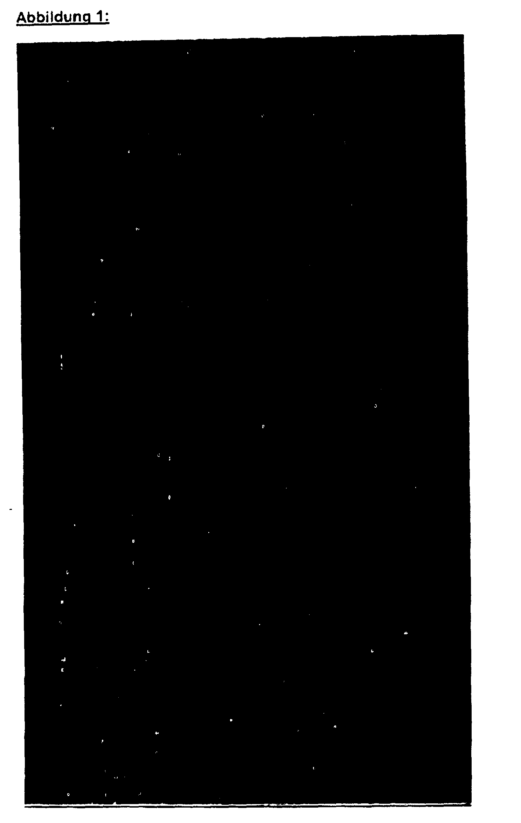

Auch ist durch die gleichmäßige Oberfiächenverteilung der die Seite (3) bildenden Fasern eine sehr gleichmäßige Verklebung des erfindungsgemäßen Hüllvlieses (1) mit dem Saugkörper (4) die Folge. Die Abbildungen 1-3 verdeutlicht dies.

Abbildung 1 zeigt das Vergleichsbeispiel 1: ein Hüllvlies aus einem thermisch - kalanderverfestigten Stapelfaser-Vliesstoff nach dem Stand der Technik, bestehend aus 100% PE/PET Bikomponentenfasern einer Feinheit von 2.2dtex, entsprechend einem Durchmesser von 14µm und mit einem Flächengewicht von 14 g/qm. Die Pressfläche des Kalanders betrug dabei 22%.

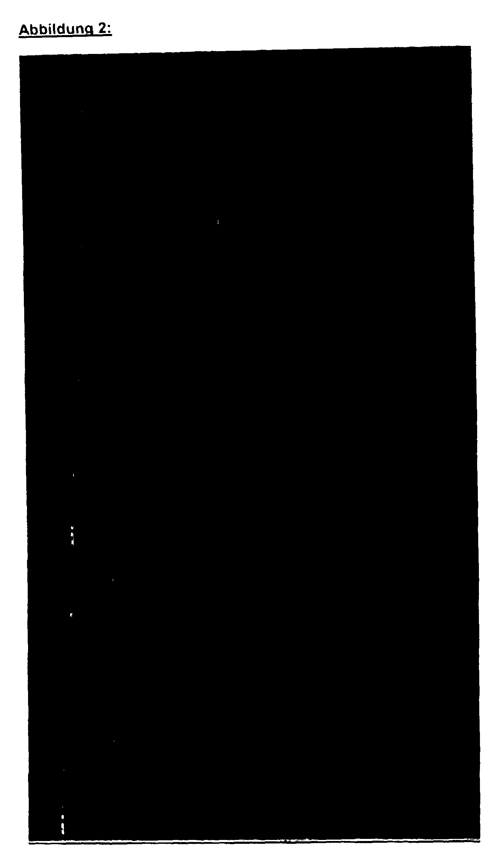

Abbildung 2 zeigt das Vergleichsbeispiel 2: ebenfalls ein Hüllvlies aus einem thermisch - kalanderverfestigten Stapelfaser-Vliesstoff nach dem Stand der Technik, bestehend aus 100% PE/PET-Bikomponenten-Fasern einer Feinheit von 2.2dtex, entsprechend einem Durchmesser von 14µm und einem Flächengewicht von 14 g/qm. Allerdings betrug hier die Pressfläche bei der Verfestigung 100%.

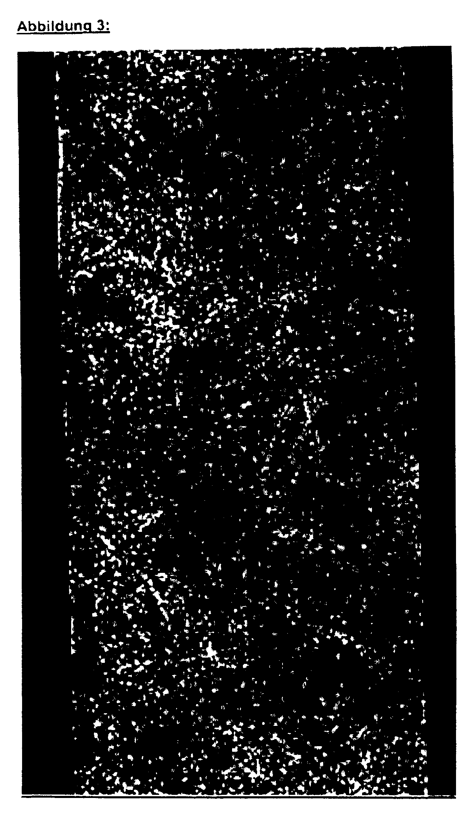

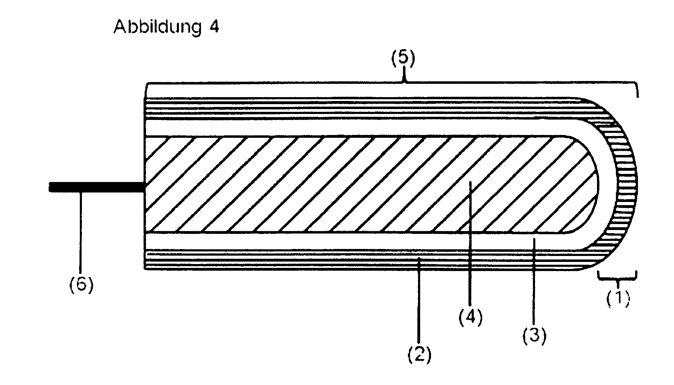

Abbildung 3 zeigt nun ein erfindungsgemäßes Hüllvlies (1), wobei die Seite (2) aus einem 12g/qm schweren Stapelfaservilesstoff, bestehend aus 100% Polyethylen / Polyethylenterephthalat- Bikomponentenfasern und deren Durchmesser bei 14µm liegt, gebildet wird. Die Verfestiqungsfläche betrug 22%. Die Seite (3) wird aus einem Feinfaser-Spinnvliesstoff mit einem Flächengewicht von 3 g/qm gebildet und besteht aus Polypropylen-Granulat mit einem Schmelzilussindex von ca 1100g/10min. Der mittlere Faserdurchmesser der die Seite (3) bildenden Fasern liegt bei 3µm.

Wie man aus den vorerwähnten Abbildungen 1 bis 3 erkennen kann, erscheint das erfindungsgemäße Hüllvlies (1) aus Abbildung 3 deutlich gleichmäßiger bei der Beurteilung der Faserverteilung. Diese uniforme Verteilung der die Seite (3) bildenden Fein-Fasern begünstigt die Verklebung zwischen der Seite (3) und dem Saugkörper (4). Man erreicht über die Breite und Länge des Verbindungsbereiches der Seite (3) mit dem Saugkörper (4) eine gleichbleibende Verbindungskraft die letztendlich auch durch die im späteren Gebrauch auftretende Quellung des Saugkörpers nur unwesentlich beeinflusst wird.

Dieses Verhalten eines unter Verwendung des erfindungsgemäßen Hüllvlieses (1) hergestellten Tampons (5) stellt eine wesentliche Verbesserung der Gebrauchssicherheit im Vergleich zu Tampons nach dem Stand der Technik dar. Tabelle 1 verdeutlicht dies nochmals.

Bei den der Tabelle 1 zugrundeliegenden Versuchen wurde mit einem Saugkern aus einem Kardenband, bestehend 80% Viskosefasern, 1,7dtex / 40mm Stapellänge und 20 % Viskose trilobal 3.3 dtex / 40mm Stapellänge gearbeitet. Das Kardenband hatte dabei eine Breite von 50mm und ein Längengewicht von 8 g/m. Als Saugkern wurde jeweils ein 50mm breites und 250mm langes Stück Kardenband eingesetzt.

Die Verpressung des Hüllvlieses mit dem Saugkern fand in einer Ruggli CL 3 Tamponmaschine bei einer Temperatur von 190°C und einem Ausstoß von 120 Tampons / Minute statt. Zum Einsatz kamen übliche Pressbacken.

Man erkennt deutlich, dass die Haftkraft zwischen Saugkern und Hüllvlies bei Verwendung des erfindungsgemäßen Hüllvlieses (1) mit einer mittleren Haftkraft von 18.47N/50mm auf einem wesentlich höheren Niveau im Vergleich zu Materialien nach dem Stand der Technik liegt.

Diese Verbesserung bleibt auch bei der Anwendung erhalten. In Tabelle 2 wird nochmals die Haftkraft zwischen Hüllvlies und Saugkern nach Quellung bewertet.

Dazu wurden die Tampons mit erfindungsgemäßem Hüllvlies und Tampon nach dem Stand der Technik für 1 Sekunde in 0,9% Kochsalzlösung getaucht und anschließend die Haftung des Hüllvlieses auf dem Saugkern gemessen.

Die Daten aus Tabelle 2 belegen, dass bei Verwendung erfindungsgemäßer Hullvlese (1) in Tampons die Haftung zwischen Hülle und Saugkern entscheidend verbessert wird. Die Aufnahmekapazität wird dabei im Übrigen nicht negativ beeinflusst, sondern bleibt auf vergleichbarem Niveau.

Ein weiterer wichtiger Aspekt bei der Beurteilung des erfindungsgemäßen Materials im Vergleich zu den aus dem Stand der Technik bekannten Materialien ist die Verhinderung von Fasermigration aus dem Saugkörper (4) heraus.

Dabei wurde die Luftdurchlässigkeit gemäß DIN 53887 bei 100Pa Differenzdruck von erfindungsgemäßem Hüllvlies (1) im Vergleich zum Stand der Technik gemessen.

Materialien nach dem Stand der Technik haben eine wesentlich höhere Luftdurchtässigkoit. Bei Hüllvliesstoffen nach dem Stand der Technik ist es daher unvermeidbar, dass abstehende Fasern des Saugkerns bedingt durch die hohe Porosität bereits während des ersten Kontakts mit dem Kardenband durch die Poren des Hüllvliesstoffes Fasern migrieren und in späteren Gebrauch auch in der Körperhöhle verbieiben können. Durch die geschlossene Oberfläche der Seite (3) des erfindungsgemäßen Hüllvlieses (1) wird dies vermieden. Die die Seite (3) bildenden Fasern haben einen wesentlich geringeren Faserdurchmesser als die im den späteren Saugkörper (4) bildenden Kardenband verwendeten Fasern.

Somit wird bereits bei der Herstellung des Tamponrohlings Fasermigration aus dem Saugkörper heraus an die Oberfläche des Tampons vermieden. Durch den Einsatz von Polymeren mit hohem Schmelzflussindex wird im erfindungsgemäßen Material darüber hinaus an der Grenzschicht des Saugkörpers (4) zum Hüllvlies (1) legendes Fasermaterial gleichmäßig mit abgebunden und kann somit nicht mehr migrieren.

In der Folge wird die Oberflächenglätte der Außenseite eines Tampons unter Verwendung eines erfindungsgemäßen Hüllvlieses (1) und somit auch der Komfort beim Einführen aber auch beim Rückholen verbessert. Diese Eigenschaft wird Tabelle 4 dargestellt.

Dabei wurden unter Verwendung der vorerwähnten Materialien hergestellte Tampons mit erfindungsgemäßem Hüllvlies (1) und Hüllvliesen nach dem Stand der Technik in ein handelsübliches Rohr aus Polycarbonat mit einem Durchmesser von 14mm und einer Länge von 100mm gesteckt. Die Tampons haben einen Durchmesser von 12mm und eine Länge von 50mm.

Die Tampons befinden sich dabei mit der Spitze an der Öffnung auf einer Seite des Rohres, sodass eine Benetzung der Spitze mit 0.9%iger Kochsalzlösung möglich ist, der Rückholfaden ragt aus der anderen Öffnung des Rohres.

Die Tamponspitze wird mit 6ml 0,9%iger Kochsalzlösung beaufschlagt, sodass der Tampon quillt. Nach einer Wartezeit von 3 Minuten wird der Tampon mit einer Geschwindigkeit von 100mmlmin am Rückholfaden aus dem Rohr gezogen, die dabei ermittelte maximale Kraft wird als Rückholkraft bezeichnet und in Newton (N) angegeben.

Betrachtet man die Tabelle 4 so zeigt sich, dass bei Verwendung des erfindungsgemäßen Hüllvlieses (1) die Auszugskraft 20% geringer ist wie bei Hüllvliesen nach dem Stand der Technik.

Die Gebrauchssicherheit eines Tampons, der unter Verwendung des erfindungsgemäßen Hüllvlieses (1) hergestellt wurde, liegt daher deutlich über den aus dem Stand der Technik bekannt Materialien.

## Patentansprüche

1. Hüllvlies (1) für absorbierende Tampons, umfassend eine im späteren Gebrauch dem Körper zugewandte Seite (2) und eine im späteren Gebrauch einem Saugkörper (4) zugewandten Seite (3)
**dadurch gekennzeichnet, dass**
die Seite (2) aus einem nach einem Trockenverfahren gebildeten Vliesstoff besteht und dass die
die Seite (3) aus einem Exlrusionsvliesstoff besteht.

2. Hullvlies (1) nach Anspruch 1,
**dadurch gekennzeichnet, dass**
die Seite (2) aus einem nach einem Trockenverfahren gebildeten Extrusionsvliesstoff besteht

3. Hüllvlies (1) nach Anspruch 1,
**dadurch gekennzeichnet, dass**
die Seite (2) aus einem nach einem Trockenverfahren gebildeten Faservliesstoff besteht

4. Hüllvlies (1) nach Anspruch 1,
**dadurch gekennzeichnet, dass**
die Seite (2) aus einem nach einem Trockenverfahren gebildeten Filamentspinnvliesstoff besteht.

5. Hüllvlies (1) nach einem der Ansprüche 1-4
**dadurch gekennzeichnet, dass**
die Seite (3) aus einem nach einem Trockenverfahren gebildeten Feinfaserspinnvliesstoff besteht

6. Hüllvlies (1) nach einem der Ansprüche 1-5,
**dadurch gekennzeichnet, dass**
die Seite (2) aus thermoplastischen Polymeren besteht.

7. Hüllvlies (1) nach einem der Ansprüche 1-5,
**dadurch gekennzeichnet, dass**
die Seite (2) aus einer Mischung von Stapelfasern aus thermoplastischen und nicht thermoplastischen Polymeren besteht.

8. Hülivlies (1) nach einem der Ansprüche 1-7,
**dadurch gekennzeichnet, dass**
das Flächengewicht des Hüllvlieses (1) in einem Bereich von 5 bis 30 g/qm hegt

9. Hüllvlies (1) nach einem der Ansprüche 1-8,
**dadurch gekennzeichnet, dass**
das Flächengewichtsverhältnis der Seite (2) zu Seite (3) maximal 10 zu 1 und minimal 1 zu 1 beträgt.

10. Hüllvlies (1) nach einem der Ansprüche 1-9,
**dadurch gekennzeichnet, dass**
der Faserdurchmesser der die Seite (2) bildenden Fasern und/oder Filamente mindestens 10µm beträgt.

11. Hüllvlies (1) nach einem der Anspruche 1-10,
**dadurch gekennzeichnet, dass**
der Faserdurchmesser der die Seite (3) bildenden Fasern maximal 10µm beträgt.

12. Tampon für Hygienezwecke bestehend aus einem Hüllvlies, einem Saugkern und einem Rückholfaden,
**dadurch gekennzeichnet, dass**
das Tamponcover aus einem Hüllvlies (1) gemäß der Ansprüche 1 - 11 gebildet wird.
